# EUROPÄISCHE PATENTSCHRIFT

(11) **EP 1 246 571 B1**
(45) Veröffentlichungstag und Bekanntmachung des Hinweises auf die Patenterteilung: **17.09.2003**
(21) Anmeldenummer: 00990823.7
(22) Anmeldetag: 27.12.2000
(51) Int. Cl.: A61B 17/02

(54) **CHIRURGISCHES INSTRUMENT**
SURGICAL INSTRUMENT
INSTRUMENT CHIRURGICAL

(30) Priorität: 28.12.1999 DE 19963456
(43) Veröffentlichungstag der Anmeldung: 09.10.2002
(73) Patentinhaber: Uttikal, Markus, 73072 Donzdorf (DE); Dunder, Thomas, 73037 Göppingen (DE)
(72) Erfinder: Uttikal, Markus, 73072 Donzdorf (DE); Dunder, Thomas, 73037 Göppingen (DE)
(74) Vertreter: Fürst, Siegfried
(86) Internationale Anmeldenummer: EP0013275
(87) Internationale Veröffentlichungsnummer: WO01047423

(56) Entgegenhaltungen:
- DE-A- 2 455 625
- US-A- 3 857 386
- US-A- 4 934 352
- US-A- 4 995 875
- US-A- 5 217 463
- US-A- 5 352 220
- US-A- 5 558 621

## Beschreibung

Die Erfindung betrifft ein chirurgisches Instrument, das als Wund- und Knochenhaken ausgebildet ist und in der Medizintechnik insbesondere für chirurgische Arbeiten bei komplizierten Frakturen einsetzbar ist.

In der Medizintechnik, insbesondere für chirurgische Arbeiten sind Wund- und Knochenhaken üblich und bekannt, die als Einzinkerhaken ausgebildet sind.
In der Operationspraxis ist es aber vielfach erforderlich, eine große Anzahl unterschiedlicher Instrumente zum Ziehen, Hebeln und Klopfen zu verwenden, wobei ein Instrumentenwechsel für einen Chirurgen abhängig von den jeweiligen Operationssituationen und Einsatzbedingungen der unterschiedlichen Instrumente sehr zeit- und kraftaufwendig ist, da es durchaus erforderlich sein kann, ein verwendetes chirurgisches Instrument mit einer anderen Handstellung zu bedienen, als dafür vorgesehen. Außerdem muss sich ein Chirurg durch operationsbedingte Handhabungen auf unterschiedlichste ergonomisch gestaltete Instrumente einstellen, was ebenfalls hohe Fertigkeiten verbunden mit Kraftaufwand erfordert.

Die US 4,934,352, der zum Oberbegriff des Anspruchs korrespondiert offenbart in Figur 12 ein chirurgisches Instrument 110 mit einem Handgriff 118, wobei der Handgriff 118 in einer Öffnung 47 mit Hilfe einer Flügelmutter 48 unverlierbar einsetzbar ist.

Der Erfindung liegt daher die Aufgabe zugrunde, einen Wundund Knochenhaken so auszubilden, dass er für unterschiedlichste chirurgische Arbeiten universell einsetzbar ist.

Zur Lösung dieser Aufgabe wird vorgeschlagen, dass das chirurgische Instrument die Merkmale des Patentanspruches 1 aufweist, insbesondere daß die Öffnung im Schaft des Instrumentes als Kegelbohrung ausgebildet ist. Das Instrument ist zweiteilig ausgebildet, indem dessen Schaft eine Öffnung aufweist, in die ein Griffelement unverlierbar einsetzbar ist, mittels dem das als Wund- und Knochenhaken ausgebildete chirurgische Instrument bei Gebrauch funktionell unterschiedlich zu handhaben ist.
Die zweiteilige Ausführungsform des Wund- und Knochenhakens ist dabei nicht an eine spezielle Form und Abmaße gebunden, sondern allgemein auf alle Hakenformen übertragbar, so dass auch die beispielsweise seit Jahrzehnten bewährten Einzinkerhaken durch Einarbeiten einer Öffnung im Schaftbereich ohne großen Aufwand nachgerüstet werden können.
Durch die zweiteilige Ausführung des Wund- und Knochenhakens kann außerdem während einer Operation durch Einsetzen des Griffelementes der Wund- und Knochenhaken mit nur einem Handgriff in kürzester Zeit in ein stabiles, handliches Werkzeug umgerüstet werden, das als Klopf-, Zieh- oder Drehwerkzeug die Arbeit eines Chirurgen bei komplizierten Frakturen erleichtert.

Es ist vorgesehen, dass diese Öffnung mittig und quer zur Längsrichtung im Schaft des chirurgischen Instrumentes angeordnet ist.

Vorteilhaft ist, dass das Griffelement als Bolzen ausgebildet ist.

Eine vorteilhafte Variante wird noch darin gesehen, dass das Griffelement in die Kegelbohrung im Schaft des chirurgischen Instrumentes mittels eines kegelförmigen Ansatzes einsteckbar ist.

Bei einer weiteren bevorzugten Ausführungsvariante ist vorgesehen, dass das Griffelement mittels eines als Kugelrast, bestehend aus einer Kugel mit einer Feder, ausgebildeten Halteelementes gehalten ist.

Die Kugel rastet dabei an der Seitenfläche des Schaftes des chirurgischen Instrumentes ein. Durch Eindrücken der Kugel kann das Halteelement ausrasten und das Griffelement in einfachster Weise wieder herausgenommen werden.

Eine vorteilhafte Ausführungsform besteht noch darin, dass der Umfang des Griffelementes, also dessen äußere Mantelfläche, im gleichen Abstand zueinander Vertiefungen aufweist, zur besseren Handhabung des Wund- und Knochenhakens bei einer Operation.

Ausführungsbeispiele der Erfindung sind in den Zeichnungen weitestgehend schematisiert dargestellt und im folgenden näher beschrieben. Dabei zeigt:
- Fig. 1: einen Wund- und Knochenhaken in Vorderansicht;
- Fig. 2: diesen Wund- und Knochenhaken in Seitenansicht;
- Fig. 3: eine Darstellung eines Griffelementes und
- Fig.4: eine Einzelheit eines Halteelementes.

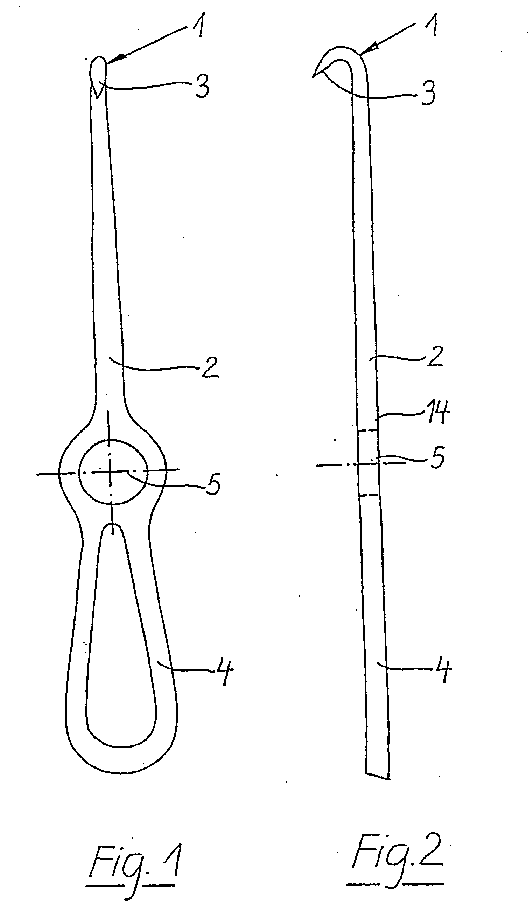

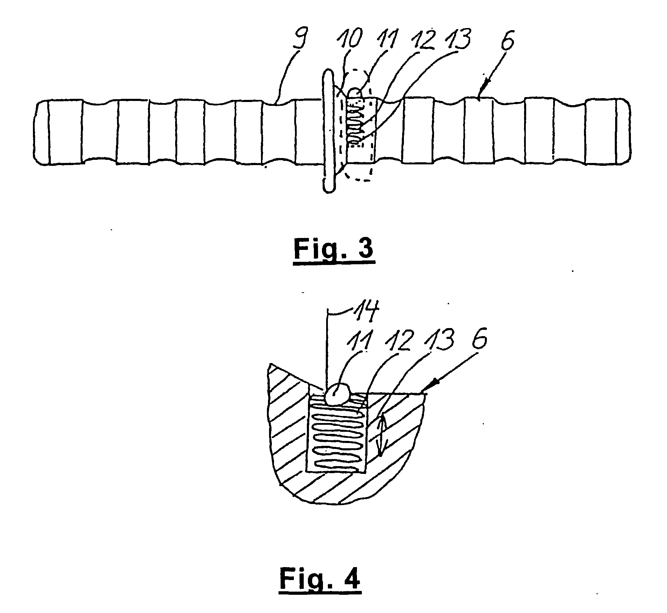

Das Beispiel in den Figuren 1 und 2 zeigt einen Wund- und Knochenhaken 1 gemäß dem Stand der Technik, der aus einem annähernd geradlinig verlaufenden Schaft 2 besteht, dessen Kopfteil als Haken 3 und dessen anderes Ende als Handgriff 4 ausgebildet ist.
Vorzugsweise im Grenzbereich zwischen dem Haken 3 und dem Handgriff 4, im wesentlichen in etwa der Mitte des Schaftes 2, ist eine kreisrunde Öffnung 5 zur Befestigung eines Griffelementes 6 quer zur Längsachse des Schaftes 2 des Wund- und Knochenhakens 1 angeordnet.
Das Griffelement 6 ist entsprechend der Figur 3 vorzugsweise bolzenförmig ausgebildet.

Der Querschnitt des Griffelementes 6 ist über dessen gesamte Körperlänge im wesentlichen gleich; jedoch sind bevorzugt an seinem Umfang, also in dessen Mantelfläche, Vertiefungen 9 vorgesehen, die im wesentlichen in gleichmäßigem Abstand zueinander angeordnet sind. Diese Vertiefungen dienen zur besseren Handhabung des Griffelementes 6, beispielsweise während einer Operation.

In einer Ausgestaltung (Figur 3) ist am Griffelement 6 ein kegelförmiger Ansatz 10 vorgesehen, der in die korrespondierende kegelförmig gestaltete Ausführung der Öffnung 5 des Wund- und Knochenhakens 1 einsetzbar ist. Die Sicherung des

Griffelementes 6 in der eingesteckten Lage erfolgt dabei mittels eines Halteelementes, das aus einer Kugel 11 und aus einer Feder 12 besteht, die in einer in dem Griffelement 6 vorgesehenen Querbohrung 13 angeordnet sind. Nach dem Einsetzen des kegelförmigen Ansatzes 10 in die kegelförmige Öffnung 5 rastet die Kugel 11, bewegt durch die Kraft der Feder 12, hinter die Seitenflächen 14 des Schaftes 2 des Wund- und Knochenhakens 1 und sichert somit die Lage des Griffelementes 6. Durch Hineindrücken der Kugel 11 gegen die Feder 12 in die Querbohrung 13 kann das Griffelement 6 dann wieder aus dem Wund- und Knochenhaken 1 herausgenommen werden.

## Patentansprüche

1. Chirurgisches Instrument (1), das als Wund- und Knochenhaken (1) ausgeführt ist und im Bereich der Medizintechnik für chirurgische Arbeiten insbesondere bei komplizierten Frakturen einsetzbar ist, umfassend einen Schaft (2) mit einem hakenförmig (3) ausgebildeten Kopfteil und einem Endteil in Form eines Handgriffes (4), wobei im Schaft (2) des zweiteiligen chirurgischen Instrumentes (1) eine Öffnung (5) vorgesehen ist, zur Aufnahme eines Griffelementes (6),
wobei die Öffnung (5) mittig und quer zu seiner Längsrichtung im Schaft (2) des chirurgischen Instrumentes (1) angeordnet ist und in die Öffnung (5) ein Griffelement (6) unverlierbar einsetzbar ist,
**dadurch gekennzeichnet, dass**
die Öffnung (5) im Schaft (2) des chirurgischen Instrumentes (1) als Kegelbohrung ausgebildet ist.

2. Chirurgisches Instrument nach Anspruche 1,
**dadurch gekennzeichnet, dass**
das Griffelement (6) bolzenförmig ausgebildet ist.

3. Chirurgisches Instrument nach Anspruch 2,
**dadurch gekennzeichnet, dass**
das Griffelement (6) in die Öffnung (5) im Schaft (2) des chirurgischen Instrumentes (1) mittels eines kegelförmigen Ansatzes (10) einsteckbar ist.

4. Chirurgisches Instrument nach Anspruch 2,
**dadurch gekennzeichnet, dass**
das Griffelement (6) mittels eines als Kugelrast (11, 12, 13) ausgebildeten Halteelementes an der Seitenfläche (14) des Schaftes (2) des chirurgischen Instrumentes (1) gehalten ist.

5. Chirurgisches Instrument nach einem der Ansprüche 1 bis 4,
**dadurch gekennzeichnet, dass**
der Umfang des Griffelementes (6) in gleichem Abstand zueinander angeordnete Vertiefungen (9) aufweist.

## Claims

1. Surgical instrument (1), which is executed as a wound and bone hook (1) and which is usable in the field of medicine for surgical work, particularly for complicated fractures, comprising a shank (2) with a head part, which is constructed to be hook-shaped (3), and an end part in the form of a handle (4), wherein an opening (5) for receiving a grip element (6) is provided in the shank (2) of the two-part surgical instrument (1), the opening (5) is arranged centrally and transversely to the longitudinal direction thereof in the shank (2) of the surgical instrument (1) and a grip element (6) is captively insertable in the opening (5), **characterised in that** the opening (5) in the shank (2) of the surgical instrument (1) is formed as a taper bore.

2. Surgical instrument according to claim 1, **characterised in that** the grip element (6) is formed to be pin-shaped.

3. Surgical instrument according to claim 2, **characterised in that** the grip element (6) is insertable into the opening (5) in the shank (2) of the surgical instrument (1) by means of a conical protrusion (10).

4. Surgical instrument according to claim 2, **characterised in that** the grip element (6) is retained by means of a retaining element, which is constructed as a ball detent (11, 12, 13), at the side surface (14) of the shank (2) of the surgical instrument (1).

5. Surgical instrument according to one of claims 1 to 4, **characterised in that** the circumference of the grip element (6) has depressions (9) arranged at the same spacing relative to one another.

## Revendications

1. Instrument chirurgical (1), qui est conçu sous forme de crochet pour plaie et pour os (1) et qui est susceptible d'être utilisé dans le domaine de la technique médicale pour les interventions de chirurgie en particulier dans le cas de fractures complexes, comprenant une tige (2), avec une tête conçue en forme de crochet (3) et une extrémité en forme de poignée (4), un orifice (5) destiné à recevoir un élément de prise (6) étant prévu dans la tige (2) de l'instrument chirurgical (1) réalisé en deux parties, ledit orifice (5) étant réalisé dans la tige (2) au milieu de l'instrument chirurgical (1) et transversalement à la direction longitudinale de celui-ci et un élément de prise (6) pouvant être inséré de manière imperdable dans l'orifice (5), **caractérisé en ce que** l'orifice (5) est réalisé sous forme de forure conique dans la tige (2) de l'instrument chirurgical (1).

2. Instrument chirurgical selon la revendication 1, **caractérisé en ce que** l'élément de prise (6) est conçu sous forme de goujon.

3. Instrument chirurgical selon la revendication 2, **caractérisé en ce que** l'élément de prise (6) est destiné à être inséré au moyen d'une saillie conique (10) dans l'orifice (5) réalisé dans la tige (2) de l'instrument chirurgical (1).

4. Instrument chirurgical selon la revendication 2, **caractérisé en ce que** l'élément de prise (6) est maintenu au moyen d'un élément de retenue, conçu sous forme de taquet sphérique (11, 12, 13), au niveau de la face latérale (14) de la tige (2) de l'instrument chirurgical (1).

5. Instrument chirurgical selon l'une quelconque des revendications 1 à 4, **caractérisé en ce que** la périphérie de l'élément de prise (6) comporte des creux (9) disposés à égale distance les uns des autres.
